# EUROPEAN PATENT APPLICATION

(11) **EP 3 695 883 A1**
(43) Date of publication of application: **19.08.2020**
(21) Application number: 19156940.9
(22) Date of filing: 13.02.2019
(51) Int. Cl.: A61P 3/04, C07K 16/18

(54) **MEANS FOR SPECIFICALLY ELIMINATING PERILIPIN-1 FRAGMENT PRESENTING ADIPOCYTES**

(71) Applicant: Alytas Therapeutics GmbH, 07745 Jena (DE)
(72) Inventor: SCHMIDT, Martin, 07743 Jena (DE); LEMKE, Cornelius Alfred Rudolf, 07743 Jena (DE); BAUMANN, Ulrich-Eckehard, 07751 Jena (DE)
(74) Representative: Engelhard, Markus

(57) **Abstract**

The present invention relates to an antigen-binding peptide specifically binding to at least one perilipin-1 epitope as defined herein. The present invention further relates to an antigen-binding peptide for use in the prevention or treatment of obesity. Furthermore, the present invention relates to a bispecific antigen-binding peptide comprising a first binding site specifically binding to a perilipin-1 epitope and a second binding site, and use thereof. The present invention also relates to a pharmaceutical composition, comprising an antigen-binding peptide or a bispecific antigen-binding peptide, for use in the prevention or treatment of obesity. The present invention further relates to an isolated nucleic acid encoding for an antigen-binding peptide or a bispecific antigen-binding peptide. Furthermore, the present invention relates to a recombinant cell comprising said nucleic acid and to a method of producing a means for specifically targeting perilipin-1 fragment presenting adipocytes.

## Description

### FIELD OF THE INVENTION

The present invention relates to an antigen-binding peptide specifically binding to at least one perilipin-1 epitope as defined herein. The present invention further relates to an antigen-binding peptide for use in the prevention or treatment of obesity. Furthermore, the present invention relates to a bispecific antigen-binding peptide comprising a first binding site specifically binding to a perilipin-1 epitope and a second binding site, and use thereof. The present invention also relates to a pharmaceutical composition, comprising an antigen-binding peptide or a bispecific antigen-binding peptide, for use in the prevention or treatment of obesity. The present invention further relates to an isolated nucleic acid encoding for an antigen-binding peptide or a bispecific antigen-binding peptide. Furthermore, the present invention relates to a recombinant cell comprising said nucleic acid and to a method of producing a means for specifically targeting perilipin-1 fragment presenting adipocytes.

### BACKGROUND OF THE INVENTION

Obesity is a worldwide problem and according to the World Health Organization, more than 1.9 billion are overweight. People suffering from obesity are commonly predisposed to a variety of medical conditions. Surgical intervention is presently a successful therapeutic approach, however, complications often arise with subsequent gastrointestinal problems. Thus, surgical intervention is considered acceptable merely in patients, which are highly obese with high comorbidity. Pharmacotherapies that were relatively promising had to be withdrawn from the market because of serious side effect profiles. The pleiotropic nature of the signaling mechanisms involved caused unacceptable side effects. Accordingly, there is a lack of therapeutic approaches which are successful in long-term and which have negligible side effects.

The harmful long-term effects of excess body fat are widely known. However, there is also a medical condition resulting in loss of body fat, namely lipodystrophy. Although metabolic complications vary with regard to the degree of fat loss, common consequences of lipodystrophy are insulin resistance or diabetes mellitus, absence of ketosis, increased metabolic rates, hyperlipidemia, as well as hepatomegaly including fatty liver. These implications result from the absence of metabolically active adipocytes irrespective of the underlying cause of adipocyte loss, as shown in various animal models. Lipodystrophy widely gained interest as a common side effect of some anti-retroviral drugs, which cause partial redistribution of fat.

Perilipin-1 is specifically expressed in adipocytes, but not in other cells storing triacylglycerols such as hepatocytes [1]. In such other cells, other members of the lipid droplet associated proteins, such as adipophilin and TIP47, occur instead of perilipin-1. As perilipin-1 shields the lipid storage droplets in adipocytes from the activity of lipolytic enzymes, any mechanism leading to depletion of lipid storage droplet-associated perilipin-1 may result in increased lipolysis and release of lipids stored in droplets (and thereby aggravate the metabolic situation). Accordingly, a reduction of fat mass is expected at the expense of an unfavourable metabolic situation. Said unfavourable metabolic situation can be prevented by completely eliminating said adipocytes. Since other types of lipid storing cells do not present perilipin-1, targeting perilipin-1 for activation of an immune-effector mechanism against adipocytes may allow for specific elimination of adipocytes. WO 02/028410 A1 discloses that perilipin may be a target for regulation of body weight, muscle mass and diabetes.

The autoimmune reaction against adipocytes leads to almost complete loss of adipose tissue as observed in generalized acquired lipoatrophy (AGL) [2]. There is broad clinical evidence for an autoimmune etiology in most cases of the classical acquired lipodystrophies. In a patient with acquired generalized lipodystrophy, serum autoantibodies against adipose tissue were found [2]. Particularly, autoantibodies against perilipin-1 were found to be associated with acquired generalized lipodystrophy [3].

Accordingly, effector systems which cause fat loss in lipodystrophy patients could be used to specifically target fat tissue in patients suffering from obesity. However, such specific effector systems, which may, for example, be antibodies or antigen-binding fragments, or cytotoxic T cells, have not been provided. Particularly, despite many efforts, no adipocyte-specific epitopes have been identified so far which could potentially be targeted as specific therapeutic targets [4].

Accordingly, the present invention aims to provide means for specifically targeting and/or eliminating adipocytes, such as an antigen-binding peptide or a cytotoxic T cell. Furthermore, the present invention aims to provide a method of producing a means for specifically targeting adipocytes.

### SUMMARY OF THE INVENTION

The present invention relates to epitopes of perilipin-1, which are molecular target structures to specifically target and/or eliminate adipocytes. Particularly, the herein disclosed epitopes are target structures of the intracellular protein perilipin-1 which are presented on the surface of adipocytes. Antigen-binding peptides that target the herein disclosed epitopes can be used alone or in combination with any therapeutic agent to target and/or eliminate adipocytes by activating an immune effector system, such as an exogenous effector system or an endogenous effector system. The herein disclosed epitopes are also useful for generating perilipin-1 specific cytotoxic T cells, which are capable of targeting perilipin-1 fragment presenting adipocytes.

The present inventors disclose perilipin-1 as the autoantigen targeted by autoantibodies from the serum of a patient with AGL. Furthermore, the present inventors disclose six epitopes of perilipin-1 having SEQ ID Nos. 1-6 and provide antibodies and antigen-binding fragments targeting said epitopes which are useful in the treatment of obesity.

The present invention further relates to producing a means to specifically target adipocytes. These means to specifically target adipocytes are useful in treating or preventing a disease and/or a medical condition related to obesity.

The present invention aims at using an exogenous or endogenous effector system of the immune system to specifically target and/or eliminate adipocytes. Such an exogenous effector system may be, for example, ex vivo or in vitro generated and/or amplified antibodies and/or cytotoxic T cells. The herein disclosed epitopes of perilipin-1, which are SEQ ID NOs. 1-6, allow for specifically targeting adipocytes via perilipin-1 fragments presented on said adipocytes. Targeting said adipocytes via perilipin-1 using an antigen-binding peptide specific for any of SEQ ID NOs. 1-6 marks said adipocytes for an effector system such as the complement system, which results in the activation of the complement system, and/or Fc-receptors, such as FcRIII (CD16), Fc-RII (CD32), Fc-RI (CD64), or others, which results in the recruitment and activation of effector systems such as ADCC (antibody dependent cellular cytotoxicity) by NK (natural killer) cells or ADCP (antibody dependent cellular phagocytosis) by macrophages, neutrophils, or any other effector system carrying the respective Fc-receptor. The recruitment of one of those effector systems leads consequently to the targeting and/or elimination of said adipocytes. Alternatively, said adipocytes can be directly targeted by cytotoxic T cells specific for perilipin-1, particularly specific for any of SEQ ID NOs. 1-6, which then eliminate said adipocytes presenting at least one of those perilipin-1 epitopes. Accordingly, a means to specifically target and/or eliminate an adipocyte according to the present invention may either directly eliminate said adipocyte, in case of ADCP, and thus be a direct elimination means, or may indirectly eliminate said adipocyte by targeting said adipocyte resulting in an activation of an effector system such as the complement system or ADCC or cytotoxic T cells, and thus be an indirect elimination means.

The cell type specificity of the effector reaction is secured by the specificity of the cellular antigen, namely perilipin-1, particularly the epitopes having an amino acid sequence according to any of SEQ ID NOs. 1-6. Accordingly, the present invention provides a means to specifically target and/or eliminate adipocytes without off-target effects on other cells, and thus with only negligible side effects.

Furthermore, the targeting of adipocytes for an immune effector system using an antigen-binding peptide allows for systemic application, e.g. by an infusion, and/or allows for fine-tuned, targeted administration to eliminate certain fat depots by administration into the respective fat depot, such as by injection into a target fat depot.

The present invention uses the adipocyte specific presentation of a fragment of a typically intracellular protein on the surface of an adipocyte, namely a perilipin-1 fragment, perilipin-1 being essential for storage of lipids in adipocytes. The present inventors disclose herein six peptide sequences which are epitopes on perilipin-1, which were identified by analyzing the binding pattern of an anti-perilipin-1 autoantibody of a patient having lipoatrophy. The plurality of the identified epitopes is highly conserved in the perilipin-1 sequences of mice and humans.

The target antigen epitopes of perilipin-1 disclosed herein allow for
a) immunizing an animal with said target structures to produce polyclonal or monoclonal antibodies and/or antigen-binding fragments against perilipin-1 peptides presented by adipocytes,
b) screening a recombinant antibody library and/or small molecule libraries to identify specific antibodies, antigen-binding fragments, or other suitable affinity ligands against perilipin-1 peptides presented by adipocytes, and/or
c) isolating and/or generating cytotoxic T cells in vitro, which are specific for perilipin-1 peptides presented by adipocytes.

With the antibodies, antibody fragments, or other suitable affinity ligands produced by a method in accordance with a) or b), it is possible to induce a specific immune effector reaction by marking said adipocytes in vivo. Furthermore, with the cytotoxic T cells produced by a method in accordance with c), adipocytes are indirectly eliminable in vivo.

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to an antigen-binding peptide specifically binding to at least one perilipin-1 epitope selected from the following amino acid sequences: LTLLDGDLPE (SEQ ID NO. 1), EKIASELK (SEQ ID NO. 2), VPIASTSDKV (SEQ ID NO. 3), PAPGHQQAQK (SEQ ID NO. 4), PSLLSRVGALTN (SEQ ID NO. 5), and PWLHSLAA (SEQ ID NO. 6).

In one embodiment, said antigen-binding peptide binds to more than one of SEQ ID NO. 1-6. In one embodiment, said perilipin-1 epitope is a human perilipin-1 epitope.

In one embodiment, said antigen-binding peptide is not a perilipin-1 targeting autoantibody of a human patient having lipoatrophy, and wherein preferably said antigen-binding peptide is not a perilipin-1 targeting anti-PLINl antibody directed to the peptide spanning Thr8-Ala145 having catalog # AF6615 from manufacturer R&D.

In one embodiment, said antigen-binding peptide competes for binding with a perilipin-1 targeting autoantibody of a human patient having lipoatrophy.

In one embodiment, said antigen-binding peptide binds to perilipin-1 in a binding assay with an affinity (K_{D}) of at least 10⁻⁴ M.

In one embodiment, said antigen-binding peptide is a monoclonal antibody.

In one embodiment, said antigen-binding peptide is a polyclonal antibody.

In one embodiment, said antigen-binding peptide is a human antibody, a humanised antibody, or a chimeric-human antibody.

In one embodiment, said antigen-binding peptide is an intact antibody, a substantially intact antibody, a Fab fragment, a F(ab')₂ fragment or a single chain Fv fragment.

In one embodiment, said antigen-binding peptide is capable of binding to a perilipin-1 fragment presented on an adipocyte, and said binding of said antigen-binding peptide to a perilipin-1 fragment on said adipocyte activates an immune effector system to target and/or eliminate said adipocyte.

In one embodiment, said antigen-binding peptide comprises an Fc region, preferably an Fc region which is optimized by protein engineering for higher binding affinity to an effector cell compared to a binding affinity of an Fc region which has not been optimized.

In one embodiment, said antigen-binding peptide does not cross-react with any other perilipin protein, such as perilipin-2 -3, -4, or -5.

In a further aspect, said antigen-binding peptide is an antigen-binding peptide for use in the prevention or treatment of obesity.

Said antigen-binding peptide is as defined above.

In a further aspect, the present invention relates to a bispecific antigen-binding peptide comprising
(i) a first binding site specifically binding to a perilipin-1 epitope having any of SEQ ID NO. 1-6; and
(ii) a second binding site.

In one embodiment, said second binding site binds to a perilipin-1 epitope having any of SEQ ID NO. 1-6, wherein said epitope is equal to or different from the epitope of said first binding site; to a white adipocyte-specific surface marker, for example the neutral amino acid transporter ASC-1; or to an immune cell specific receptor molecule, such as a T cell specific receptor molecule or a natural killer cell specific receptor molecule.

Said epitope is as defined in an embodiment above.

In a further aspect, said bispecific antigen-binding peptide is a bispecific antigen-binding peptide for use in the prevention or treatment of obesity.

In a further aspect, the present invention relates to a pharmaceutical composition, comprising said antigen-binding peptide and/or said bispecific antigen-binding peptide, for use in the prevention or treatment of obesity.

Said antigen-binding peptide and said bispecific antigen-binding peptide are as defined in any of the embodiments above.

In a further aspect, the present invention relates to an isolated nucleic acid encoding for said antigen-binding peptide or encoding for said bispecific antigen-binding peptide.

Said antigen-binding peptide and said bispecific antigen-binding peptide are as defined in any of the embodiments above.

In a further aspect, the present invention relates to a recombinant cell comprising said nucleic acid, wherein said recombinant cell is preferably a recombinant autologous effector cell or a recombinant host cell.

Said nucleic acid is as defined in an embodiment above.

In a further aspect, the present invention relates to a method of producing a means for specifically targeting perilipin-1 fragment presenting adipocytes, comprising:
a) Inoculating a non-human animal with an antigen comprising at least one perilipin-1 specific epitope selected from SEQ ID NO. 1-6; and screening and producing an antigen-binding peptide specifically binding to the at least one perilipin-1 specific epitope from the inoculated animal, preferably by harvesting cells expressing an antigen-binding peptide from the spleen of said non-human animal, preparing a hybridoma cell therefrom, expressing said antigen-binding peptide, and screening said antigen-binding peptide expressed by said hybridoma cell to identify an antigen-binding peptide specifically binding to any of SEQ ID NO. 1-6, and isolation thereof; or
b) providing and screening a recombinant antigen-binding peptide library, such as a library of antibodies, scFv molecules, Camel antibodies, Anticalins, or DARPins, to identify an antigen-binding peptide specifically binding to any of SEQ ID NO. 1-6, and isolation thereof; or
c) providing and screening a cell pool comprising cytotoxic T cells, such as by Tetramer-staining or Dextramer-Technology, to identify a cytotoxic T cell having specificity to any of SEQ ID NO. 1-6, and isolation thereof; or
d) providing a blood sample of a patient and obtaining T cells therefrom, genetically modifying said T cells by introducing a nucleic acid as defined above, and obtaining chimeric antigen receptor expressing T cells; or
e) providing and screening a pool of T cell receptors to identify a T cell receptor having specificity to any of SEQ ID NO. 1-6, and isolation thereof.

In one embodiment, said means targets perilipin-1 and is selected from an antigen-binding peptide and a cytotoxic T cell.

Said antigen-binding peptide and said epitopes are as defined in an embodiment above.
In a further aspect, the present invention also relates to the use of an antigen-binding peptide or a bispecific antigen-binding peptide in the manufacture of a pharmaceutical composition for the treatment or prevention of obesity.

Said antigen-binding peptide, said bispecific antigen-binding peptide, and said pharmaceutical composition are as defined above.

In a further aspect, the present invention also relates to the use of an antigen-binding peptide, a bispecific antigen-binding peptide, or a specific cytotoxic T cell in the manufacture of a preparation, preferably a pharmaceutical preparation, for the treatment or prevention of obesity.

Said antigen-binding peptide, said bispecific antigen-binding peptide, and said specific cytotoxic T cell are as defined above.

In a further aspect, the present invention also relates to a method of preventing or treating obesity comprising administering an antigen-binding peptide, a bispecific antigen-binding peptide, and/or a cytotoxic T cell, specifically binding to any of the perilipin-1 epitopes having SEQ ID Nos. 1-6, to a patient in need thereof.

In one embodiment, said patient is a human.

Said antigen-binding peptide, said bispecific antigen-binding peptide, and said cytotoxic T cell are as defined above.

In a further aspect, the present invention relates to the use of at least one perilipin-1 epitope having any of SEQ ID NOs. 1-6 for the identification of an antigen-binding peptide, a bispecific antigen-binding peptide, and/or a cytotoxic T cell that is specific for adipocytes and/or may be used in a method for the treatment or prevention of obesity.

Said antigen-binding peptide, said bispecific antigen-binding peptide, and said cytotoxic T cell are as defined above.

### DETAILED DESCRIPTION

The term "antigen-binding peptide", as used herein, relates to a peptide which specifically binds to an antigen, preferably a peptide which binds to a perilipin-1 epitope selected from any of SEQ ID NOs. 1-6. In one embodiment, an antigen-binding molecule is based on an immunoglobulin, such as a polyclonal or monoclonal antibody, or based on a protein scaffold structure having antigen-binding capacity, such as an anticalin protein, an Affilin, an Affimer, an Affitin, an Alphabody, a nanobody, or a DARPin. In one embodiment, an antigen-binding peptide relates to an antibody or an antibody fragment. In one embodiment, when referring to an antibody or antibody fragment, it is referred to an antigen-binding peptide. There are several classes of antibodies, particularly human antibodies, such as IgA, IgG, IgM, IgD and IgE, as well as subclasses, such as IgG1, IgG2, IgG3, and IgG4. An antibody is a protein produced mainly by plasma cells of the immune system to neutralize pathogens. Antibodies are grouped into classes, also referred to as isotypes, as determined genetically by the constant region. Human constant light chains are classified as kappa (Cκ) and lambda (Cλ) light chains. Human heavy chains are classified as mu, delta, gamma, alpha, or epsilon, and define the antibody's isotype as IgM, IgD, IgG, IgA, and IgE, respectively. The IgG class is the most commonly used for therapeutic purposes. "IgG", as used herein, relates to a polypeptide belonging to the class of antibodies that are substantially encoded by a recognized immunoglobulin gamma gene. In humans this class comprises subclasses IgG1, IgG2, IgG3, and IgG4. In mice this class comprises subclasses IgG1, IgG2a, IgG2b and IgG3. IgA has several subclasses, including but not limited to IgA1 and IgA2. Thus, "isotype", as used herein, relates to any of the classes or subclasses of immunoglobulins defined by the chemical and antigenic characteristics of their constant regions. The known human immunoglobulin isotypes are IgG1, IgG2, IgG3, IgG4, IgA1, IgA2, IgM, IgD, and IgE. Also useful for the invention may be IgGs that are hybrid compositions of the natural human IgG isotypes. Effector functions such as ADCC, ADCP, CDC, and serum half-life differ significantly between the different classes of antibodies, including for example human IgG1, IgG2, IgG3, IgG4, IgA1, IgA2, IgD, IgE, and IgM. In one embodiment, an antigen-binding peptide may comprise a domain of any of the above mentioned antibody classes, subclasses, and/or chains. In one embodiment, the amino acid sequence of an antigen-binding peptide may be modified by protein engineering, e.g. to comprise constant regions from other immunoglobulin classes mediating improved effector function properties. Such engineered hybrid IgG compositions may provide improved effector function properties, including improved ADCC, phagocytosis, CDC, and serum half-life.

According to the present invention, an antigen-binding peptide may relate, for example, to human antibodies, human-chimeric antibodies, humanized antibodies, and antibody fragments. An antibody recognizes an antigen via the Fab fragment variable region comprising a paratope. Said paratope specifically targets an epitope on an antigen. In one embodiment, an antigen-binding peptide according to the present invention binds to any of the epitopes having SEQ ID NOs. 1-6 on perilipin-1. In one embodiment, said binding of an antigen-binding peptide to a perilipin-1 fragment presented on the cell surface of an adipocyte results in activation of an immune effector system to eliminate said adipocyte. Furthermore, the term "antigen-binding peptide", as used herein, relates to a peptide that specifically binds to a target antigen, such as to one or more epitope(s) displayed by or present on a target antigen. The antigen bound by an antigen-binding peptide of the present invention is perilipin-1, or in the case of bispecific molecules perilipin-1 and optionally another antigen, such as an antigen on an effector cell, such as an antigen on a cytotoxic T cell, or an antigen presented on an adipocyte other than a perilipin-1 fragment, or an antigen presented by any component of fat tissue. In one embodiment, a bispecific molecule comprises a first binding site binding for one of the perilipin-1 epitopes having any of SEQ ID NO. 1-6, and a second binding site for the same perilipin-1 epitope as the first binding site or for a perilipin-1 epitope having any of SEQ ID NO. 1-6 different from the epitope of the first binding site. In one embodiment, a bispecific molecule comprises a first binding site binding for one of the perilipin-1 epitopes having any of SEQ ID NO. 1-6, and a second binding site for an antigen different from perilipin-1.

In one embodiment, an antigen-binding peptide is a TCR-derived antigen-binding peptide and relates to a T cell receptor specific for any of SEQ ID NOs. 1-6, or to a fragment of a T cell receptor specific for any of SEQ ID NOs. 1-6. In one embodiment, said T cell receptor may be a soluble T cell receptor or may be expressed on the surface of a genetically modified cell, preferably a T cell. In one embodiment, said TCR-derived antigen-binding peptide is used for an adoptive T cell receptor transfer. In one embodiment, an antigen binding peptide relates to a peptide expressed on the surface of a genetically modified cell, particularly a T cell or NK cell, wherein said genetically modified cell is genetically modified to express said antigen-binding peptide, resulting in a CAR-T cell or CAR-NK cell, respectively.

According to the present invention, an antigen-binding peptide may further relate to a fragment of an antibody, such as a substantially intact antibody, a Fab fragment, a F(ab')₂ fragment, a diabody, a single chain Fv fragment, a tetrabody, a triabody, a disulfide bond-stabilized Fv (dsFv), or a heavy chain VHH fragment from camels. However, other forms of an antigen-binding peptide are also envisioned by the present invention. For example, the antigen-binding peptide may be another (non-antibody) receptor protein derived from small and robust non-immunoglobulin scaffolds, such as those equipped with binding functions for example by using methods of combinatorial protein design. Such a non-antibody antigen-binding peptide may be, for example, an Affibody molecule, an Affilin, an Affimer, an Affitin, an Alphabody, an Anticalin, a nanobody, or a DARPin. An antigen-binding peptide may also relate to other antibody-mimetic molecules such as a dual-affinity re-targeting antibody (DART). In one embodiment, an antigen-binding peptide preferably relates to an antibody or an antibody fragment. In one embodiment, an antigen-binding peptide may relate to any antibody-mimetic molecule which mimics the binding properties of an antibody to an antigen but which is structurally distinct from a naturally occurring antibody.

In one embodiment, an antigen-binding peptide may be optimized by protein engineering methods known to a person skilled in the art, such as directed evolution, phage display, or ribosome display, to have improved properties such as higher binding affinity and longer serum half-life compared to a non-optimized antigen-binding peptide. In one embodiment, an antigen-binding peptide may be modified by protein engineering methods known to a person skilled in the art to have the capacity to induce certain effector functions, such as by comprising an Fc region to be targeted by an effector cell. In one embodiment, said optimized antigen-binding peptide may be engineered by specific amino acid exchanges in the amino acid sequence of said Fc region to increase the response of effector systems to said Fc region. In one embodiment, said optimized antigen-binding peptide may be optimized to have an improved serum half-life, for example by modification of a CH3 region of an IgG domain. In one embodiment, an antigen-binding peptide may be modified by protein engineering methods known to a person skilled in the art to improve the capacity to induce certain effector functions, such as stronger effector responses by the complement system or cytotoxic T cells when recognizing said optimized antigen-binding peptide bound to an epitope having any of SEQ ID NOs. 1-6 presented on the surface of an adipocyte compared to a non-optimized antigen-binding peptide bound to an epitope having any of SEQ ID NOs. 1-6 presented on the surface of an adipocyte. In one embodiment, an antigen-binding peptide is a bispecific antigen-binding peptide.

The terms "complementarity determining region", "CDR", and "hypervariable region", as used herein, relate to one or more of the hypervariable or complementarity determining regions (CDRs) found in the variable regions of light or heavy chains of antibodies. The CDRs in each chain are held in close proximity by framework regions and contribute to the formation of the antigen-binding site together with the CDRs from the other chain. In one embodiment, an antigen-binding peptide comprises at least one, preferably all, CDR(s) of an antibody specifically binding to a perilipin-1 fragment, preferably specifically binding to a perilipin-1 epitope having any of SEQ ID NO. 1-6. In one embodiment, an antigen-binding peptide comprises a peptide scaffold and at least one, preferably all, CDR(s) of an antibody specifically binding to a perilipin-1 fragment, wherein said peptide scaffold may be an antibody fragment or any non-antibody peptide scaffold such as an anticalin, an Affilin, an Affimer, an Affitin, an Alphabody, an a nanobody, or a DARPin.

The term "bispecific antigen-binding peptide", as used herein, relates to an artificial protein which comprises a first binding site specifically binding to a perilipin-1 epitope having any of SEQ ID NO. 1-6 and a second binding site. A bispecific antigen-binding peptide can simultaneously bind to two target structures, such as to two different types of antigens. In one embodiment, said bispecific antigen-binding peptide is immunoglobulin-based. In one embodiment, said bispecific antigen-binding peptide comprises two different immunoglobulin-based binding fragments having binding specificity to an antigen, and said two fragments are connected by a linker fragment such as a linker peptide sequence and/or an antibody scaffold structure. In one embodiment, said binding fragments of a bispecific antigen-binding peptide may be specific for different antigens, or for different epitopes on the same antigen, or for the same epitope on the same antigen. In one embodiment, a bispecific antigen-binding peptide according to the present invention binds to a first perilipin-1 epitope and to a second, but different non-overlapping perilipin-1 epitope. In one embodiment, a bispecific antigen-binding peptide according to the present invention binds to a perilipin-1 epitope and to a different antigen. In one embodiment, said different antigen is a surface marker of an effector cell, such as a T cell or a natural killer cell. In one embodiment, said different antigen may also be an antigen presented on an adipocyte other than a perilipin-1 fragment, such as a neutral amino acid transporter ASC-1. In one embodiment, said different antigen may also be an antigen presented by any component of fat tissue other than perilipin-1. In one embodiment, a bispecific antigen-binding peptide is a fusion protein comprising a TCR-derived antigen-binding peptide. In one embodiment, a bispecific antigen-binding peptide comprises a TCR-derived antigen-binding peptide as first binding site and a second binding site derived from a molecule selected from an antibody fragment, an anticalin, a DARPin, an aptamer, an Affibody molecule, an Affilin, an Affimer, an Affitin, an Alphabody, and a nanobody. In one embodiment, said second binding site binds specifically to a receptor on immune cells such as T cells or natural killer cells, preferably to receptors capable of activating the immune cell or of stimulating an immune response of the immune cell, in which the immune response is preferably a cytotoxic immune response. In one embodiment, said receptor is preferably a receptor molecule such as CD3, CD3δ/ε, CD3γ/ε, TCR, TCRα, TCRβ, CD2, CD5, CD28, OX40, 4-1BB, CD16, Ly49, NKp30 (CD337), NKp44 (CD336), NKp46 (NCR1), or CD3ζ (CD247). In one embodiment, said second binding site of the bispecific antigen-binding peptide binds specifically to a second perilipin-1 epitope having any of SEQ ID NO. 1-6, and said second perilipin-1 epitope is different from and non-overlapping with the perilipin-1 epitope of said first binding site.

The term "autoantibody", as used herein, relates to an antibody which is produced by the immune system of a patient and which is directed against one or more of the patient's own proteins. Many autoimmune diseases are caused by such autoantibodies. In one embodiment, an anti-perilipin-1 autoantibody is involved in acquired generalized lipodystrophy. In one embodiment, said anti-perilipin-1 autoantibody triggers complement mediated destruction of adipocytes in acquired generalized lipodystrophy.

The term "lipodystrophy", as used herein, relates to a medical condition in which a body is unable to produce and maintain healthy adipose tissue. There are different types of lipodystrophies, such as congenital lipodystrophy syndromes and acquired lipodystrophy syndromes. The term "Lipoatrophy" typically describes the (almost) complete loss of adipose tissue from one area of the body or from the whole body. In one embodiment, lipodystrophy is used synonymously with lipoatrophy. In one embodiment, lipodystrophy relates to acquired generalized lipoatrophy (AGL). In one embodiment, the present invention discloses an anti-perilipin-1 autoantibody involved in lipodystrophy.

The term "perilipin-1", as used herein, is also known as lipid droplet-associated protein and relates to a protein associated with the surface of lipid droplets. Perilipin-1 belongs to the family of perilipins which are a family of proteins associated with lipid droplet surfaces. Perilipin-1 expression is elevated in obese humans. Perilipin-1 protects lipid droplets from lipases involved in lipolysis. In humans, there are three perilipin-1 isoforms, namely perilipin-A, perilipin-B, and perilipin-C. The human amino acid sequence of perilipin-1 is in accordance with SEQ ID NO. 7. In one embodiment, the term "perilipin-1" relates to a full-length perilipin-1 protein and/or a fragment thereof.

The term "epitope", as used herein, relates to the part of an antigen that is recognized by the immune system, specifically by antibodies, B cells, or T cells. In one embodiment, epitope is used synonymously with antigenic determinant. The part of an antibody that binds to the epitope is a paratope. An epitope may be a conformational epitope or a linear epitope, depending on the structure and interaction with the paratope. In one embodiment, antigen perilipin-1 comprises six epitopes, wherein said epitopes have any of the sequences as disclosed in SEQ ID NOs. 1-6. An antibody or antigen-binding fragment according to the present invention may bind to one or more of SEQ ID NOs. 1-6. In one embodiment, an antibody or antigen-binding fragment according to the present invention may also bind to a sequence having at least 85% identity with any of SEQ ID NOs. 1-6.

The term "antigen", as used herein, relates to a molecule capable of inducing an immune response in an organism. An antigen may be targeted by an antibody. The antigenic determinant of an antigen is a distinct surface feature, namely an epitope. Most antigens are potentially bound by multiple antibodies, each of which is typically specific to one of the antigen's epitopes. An autoantigen is a normal protein of an organism that is recognized by the immune system of said organism suffering from a specific autoimmune disease. In one embodiment, an antigen relates to a perilipin-1 protein and/or a fragment thereof. In one embodiment, the antigen, particularly a perilipin-1 fragment, is presented by an MHC molecule on the surface of an adipocyte. In one embodiment, said MHC molecule is an MHC-I or MHC-II molecule.

The term "targeting", as used herein, relates to the capacity of a molecular structure, such as an antibody, an antigen-binding peptide, or a surface molecule of a cytotoxic T cell, to bind to a certain structure, such as an antigen, specifically an epitope, by specific interaction. In one embodiment, a perilipin-1 fragment on the surface of an adipocyte is targeted by an antigen-binding peptide, which results in activation of an effector system, which in turn results in the elimination of said adipocyte, such as, for example, via a caspase cascade. In one embodiment, a perilipin-1 fragment on the surface of an adipocyte is targeted by a cytotoxic T cell, which specifically binds to said perilipin-1, preferably resulting in cell death of said adipocyte. In one embodiment, the terms "targeting" and "recognizing" are used interchangeably. In one embodiment, an antigen-binding peptide of the present invention targets a perilipin-1 protein and/or a perilipin-1 fragment, preferably via an epitope having any of SEQ ID NOs. 1-6. In one embodiment, an antigen-binding peptide of the present invention targets a perilipin-1 fragment presented by an MHC on a cell surface. In one embodiment, an antigen-binding peptide targets at least one of the perilipin-1 epitopes having SEQ ID NOs. 1-6. In one embodiment, targeting of a perilipin-1 fragment presented on an adipocyte using an antigen-binding peptide preferably results in elimination of said adipocyte via an effector/elimination mechanism.

The term "eliminating", as used herein, relates to targeting a target cell and inducing cell destruction, for example by a complement cascade, or by recruitment of NK cells, or by attack from a cytotoxic T cell, and preferably further comprises degradation and/or removal of the resulting cell debris. In one embodiment, an antigen-binding peptide of the present invention triggers elimination of an adipocyte via an effector mechanism. In one embodiment, a target adipocyte cell presenting a perilipin-1 fragment on its surface is eliminated by induction of apoptosis via a cytotoxic T cell, or by lysis of said cell via a complement cascade. In one embodiment, the term "eliminating" may refer to damaging a cell, destroying a cell, degrading a cell, and/or phagocytosing a cell. In one embodiment, eliminating a cell refers to damaging a cell in a manner sufficient to terminate the cell's ability to survive and/or sufficient to terminate the cell's ability to maintain its cell function. In many of the embodiments, elimination preferably relates to complete removal of an adipocyte. In many of the embodiments, "eliminating" preferably relates to destruction and complete degradation of an adipocyte without remaining extracellular cell debris, preferably by phagocytosis of said adipocyte. In one embodiment, a perilipin-1 presented on an adipocyte is targeted using an antigen-binding peptide to eliminate said adipocyte. In one embodiment, elimination of an adipocyte may be triggered by one effector mechanism, by several effector mechanisms simultaneously, or by a cascade of effector mechanisms.

The term "effector mechanism", as used herein, relates to a mechanism which is involved in and/or results in the elimination of a target cell. An effector mechanism, as used herein, may relate to various mechanisms of action involved in the destruction of a target cell, such as activation of a death pathway in a target cell, blockade of necessary growth factor/growth factor receptor interaction, induction of antibody-dependent cellular cytotoxicity (ADCC), a complement-dependent cytotoxicity (CDC) response, and/or of antibody-dependent cell phagocytosis (ADCP). In one embodiment, an effector mechanism is an immune effector mechanism which is a mechanism of the immune system to eradicate cells such as infected cells. In one embodiment, "effector mechanism" is used interchangeably with "effector system". In one embodiment, the term effector mechanism may refer to an individual effector mechanism involved in eliminating an adipocyte or to a group of several effector mechanisms which are involved simultaneously or as a cascade of mechanisms in eliminating an adipocyte.

In one embodiment, the interaction of an antigen-binding peptide with complement initiates a complement-dependent cytotoxicity (CDC) response. In one embodiment, binding of an antigen-binding peptide to a perilipin-1 fragment presented on a surface of an adipocyte mediates apoptosis of said adipocyte. In one embodiment, an antigen-binding peptide comprises an Fc (fragment crystallisable)-derived region which mediates an effector function such as antibody-dependent cell-mediated cytotoxicity (ADCC), complement-dependent cytotoxicity (CDC), and/or antibody-dependent cellular phagocytosis (ADCP), preferably via interaction with Fc receptors expressed by effector cells and/or interaction with components of the complement system such as complement component 1q (C1q). In one embodiment, an antigen-binding peptide comprises an Fc region which contributes to long half-life of said antigen-binding peptide mediated by binding to the salvage receptor FcRn. In one embodiment, glycosylation of the antigen-binding peptide may alter the intensity of an effector function mediated by said antigen-binding peptide. In one embodiment, effector cells such as NK cells, monocytes, macrophages, and granulocytes recognize an antigen-binding peptide of the present invention bound to a target cell, preferably an adipocyte, said recognition being carried out for example via a receptor FcyR of said effector cell and an Fc domain of said antigen-binding peptide, and this recognition triggers effector cells to release cytoplasmic perforin, granulysin, and granzymes that induce apoptosis and/or lysis of said target cell. In one embodiment, an effector mechanism resulting in the destruction of an adipocyte may also comprise a cytotoxic drug, a toxin, or a radionuclide that is delivered to said adipocyte by an antigen-binding peptide of the present invention, such as by conjugation of said cytotoxic drug, toxin, or radionuclide to said antigen-binding peptide, or by delivering said cytotoxic drug, toxin, or radionuclide using a liposome which is labeled with an antigen-binding peptide.

In one embodiment, an effector mechanism may also involve a chimeric antigen receptor (CAR) which is a receptor protein that has been engineered to give a T cell the ability to target a specific protein. Particularly, T cells are harvested from a patient, genetically altered to comprise a chimeric antigen receptor including an antigen-binding peptide of the present invention, e.g. by transforming said T cells with a plasmid/virus comprising the genetic information for a fusion protein including an antigen-binding peptide, such as a TCR-derived antigen-binding peptide, as well as a transmembrane domain and a cytoplasmic effector domain, and then said genetically altered T cells are administered as CAR-T cells to said patient to attack adipocytes. In one embodiment, said CAR-T cells may be derived from T cells of the patient's own blood (autologous cells) or derived from T cells of another healthy individual (allogenic cells). In one embodiment, said CAR comprises an antigen-binding peptide specific for any of SEQ ID NOs. 1-6 as ectodomain, as well as a transmembrane domain and an endodomain. In one embodiment, said antigen-binding peptide specific for any of SEQ ID NOs. 1-6 may be a T cell receptor or may be a domain of a T cell receptor. In one embodiment, an effector mechanism may involve a T cell genetically altered to be specific for any of SEQ ID NOs. 1-6 and/or a T cell naturally specific for any of SEQ ID NOs. 1-6 which is selected from a pool of T cells obtained from a donor and amplified in vitro, wherein said donor may be autologous or allogenic.

The term "effector cell", as used herein, refers to a cell, preferably an immune cell, which is involved in an effector system and/or effector mechanism resulting in the destruction and/or elimination of a target cell. In one embodiment, an effector cell may be a monocyte, macrophage, neutrophil, NK cell, NKT cell, or a cytotoxic T cell. In one embodiment, an effector cell recognizes and/or binds to an antigen-binding peptide of the present invention bound to a perilipin-1 fragment presented on an adipocyte, recognizes and/or binds to a perilipin-1 fragment presented on an adipocyte, or recognizes and/or binds to a complex thereof. In one embodiment, said recognizing and/or binding induces an effector mechanism resulting in the elimination/destruction of said adipocyte. In one embodiment, an effector cell such as a T cell recognizes an adipocyte target cell and induces cell death of said target cell.

In one embodiment, an effector cell such as a NK cell, a monocyte, a macrophage, or a granulocyte recognizes an antigen-binding peptide of the present invention bound to an adipocyte, and this recognition triggers induction of apoptosis or lysis of said adipocyte, and/or phagocytosis of said adipocyte.

The term "adipocyte", as used herein, relates to a fat cell which is specialized in storing fat. Adipose tissue is primarily composed of adipocytes. There are different types of fat tissue, namely white fat, brown fat, beige fat and marrow fat, and accordingly also different types of fat cells. White fat cells are monovacuolar cells containing one large lipid droplet surrounded by a layer of cytoplasm. Brown fat cells are plurivacuolar and are typically in a polygonal shape. Beige fat comprises white fat cells and brown fat cells, which make beige fat metabolically more desirable than white fat. There are also adipocytes in marrow adipose tissue which also seemed to have a role in obesity. In one embodiment, the terms "perilipin-1 presenting adipocyte" and "perilipin-1 fragment presenting adipocyte" are used interchangeably. In one embodiment, an adipocyte preferably presents a fragment of perilipin-1 on its surface. In one embodiment, said fragment of perilipin-1 presented on a surface of an adipocyte preferably comprises any of SEQ ID NOs. 1-6.

The term "direct elimination mechanism", as used herein, relates to a process and/or a capacity of an immune effector system to directly destruct a target cell and remove its constituents. In one embodiment, a target cell is destroyed and its constituents are removed via the activation of a macrophage by Fc-receptor mediated binding of an antigen-binding peptide to perilipin-1 or fragments thereof. In one embodiment, a direct elimination mechanism involves neutrophils (neutrophilic polymorphonuclear granulocytes) by Fc-receptor mediated binding of an antibody or antigen-binding fragment to perilipin-1 or fragments thereof. In one embodiment, a direct elimination mechanism preferably comprises macrophages and/or neutrophils binding to an antigen-binding peptide bound to a perilipin-1 fragment on the surface of an adipocyte, wherein said binding results in phagocytosis of said adipocyte, wherein said binding is preferably mediated via an Fc receptor.

The term "indirect elimination mechanism", as used herein, relates to a process and/or a capacity of an immune effector system to target a target cell and involve another system to destroy said target cell and/or to remove the cell remnants. In one embodiment, an indirect elimination mechanism involves binding of an antigen-binding peptide to a target structure such as perilipin-1 or fragments thereof on the surface of an adipocyte. In one embodiment, said binding triggers destruction of said adipocyte via the activation of a complement cascade. In one embodiment, this mechanism is complement dependent cytotoxicity (CDC). In one embodiment, an indirect elimination mechanism involves antibody-dependent cell-mediated cytotoxicity (ADCC) which involves lysis of a target cell by an effector cell of the immune system, such as a natural killer cell. In one embodiment, an indirect elimination involves antibody-dependent cellular phagocytosis (ADCP) which involves phagocytosis of an adipocyte by an effector cell of the immune system, such a macrophage. In one embodiment, an indirect elimination mechanism involves cytotoxic T-cell-mediated cytotoxicity which involves lysis of an adipocyte by an effector cell which directly binds to perilipin-1-fragments presented via an MHC on that adipocyte. Said destruction of an adipocyte then triggers the activation of another system that results in elimination of the debris of said destroyed adipocyte, such as phagocytosis. In one embodiment, an indirect elimination mechanism involves phagocytosis of target cell remnants by macrophages or neutrophils (neutrophilic polymorphonuclear granulocytes). In one embodiment, a means for specifically targeting and/or eliminating adipocytes may have both a direct elimination capacity and an indirect elimination capacity. In one embodiment, macrophages and neutrophils are both a direct elimination means, such as by Fc receptor dependent binding to a target adipocyte and phagocytosis thereof, and an indirect elimination means, such as by phagocytosis of debris of destroyed adipocytes which have been targeted by a different elimination means such as complement-mediated opsonization. In one embodiment, direct elimination mechanisms and indirect elimination mechanisms may occur concurrently and/or their scope may overlap. In one embodiment, the term "effector mechanism" may refer to both indirect and direct elimination mechanisms. In one embodiment, the terms "effector mechanism" and "elimination mechanism" may be used synonymously.

The term "effector system" or "immune effector system", as used herein, relates to any system involved in the immune system that can potentially contribute to an immune response. An immune effector system may, for example, relate to the complement system, to a T cell such as a cytotoxic T cell or a regulatory T cell, to a natural killer cell, to a B cell, to a plasma cell, or to macrophage. In one embodiment, an effector system relates to the complement system, macrophages, neutrophils, NK cells, NKT cells, and/or to cytotoxic T cells. In one embodiment, an effector system is triggered by the binding of an antigen-binding peptide to perilipin-1 presented on an adipocyte, and said triggering results in the elimination of said adipocyte.

The term "endogenous effector system", as used herein, relates to an immune effector system which is endogenous. An endogenous effector system may be, for example, the complement system which is capable of triggering targeted cell destruction upon activation, such as via opsonization. An endogenous effector system may also relate to endogenous cytotoxic T cells which are activated by specific antigens. An endogenous effector system may also relate to a macrophage, or a neutrophil such a neutrophilic polymorphonuclear granulocyte, or to a NK cell.

The term "exogenous effector system", as used herein, relates to an immune effector system which is exogenous. An exogenous effector system may be, for example, cytotoxic T cells, which are amplified ex vivo and subsequently administered to a patient. Such ex vivo amplified cytotoxic T cells may either derive from said patient or from a donor, or may derive from the commercially available source. In one embodiment, said exogenous effector system may relate to a chimeric antigen receptor T cell (CAR-T cell), which is a genetically modified T cell that expresses a T cell receptor specific for any of SEQ ID NOs. 1-6. In one embodiment, an exogenous effector system may also relate to an exogenous T cell receptor (TCR), wherein said exogenous TCR may be a soluble TCR or a TCR expressed by an effector cell such as a T cell, preferably a T cell transformed to express a TCR specific for any of SEQ ID NOs. 1-6.

The term "complement system", as used herein, relates to a part of the innate immune system that enhances the ability of antibodies and phagocytic cells to clear damaged cells from an organism. More than thirty proteins are involved in the complement system. The complement system is capable of triggering different immune functions, such as phagocytosis by opsonizing antigens, inflammation by attracting macrophages and neutrophils, and membrane attack. The classical complement pathway typically requires antigen-antibody complexes for activation. The complement system may involve a membrane attack complex, which forms a transmembrane channel causing osmotic lysis of the target cell. Furthermore, macrophages help to clear complement-coated target cells.

The term "cytotoxic T cell", as used herein, relates to a T lymphocyte which is able to kill a cancer cell, an infected cell or a cell which is damaged in another way. A T cell typically expresses a T cell receptor (TCR) that specifically recognizes an antigen. Such an antigen is typically presented on the surface of a cell by an MHC-I molecule. If the T cell receptor of a cytotoxic T cell is specific for an antigen, it binds to the complex of the antigen and the MHC-I molecule and destroys the cell. The interaction of a T cell with the complex of an antigen and a MHC-I molecule of a target cell typically also involves a co-receptor, namely CD8. In one embodiment, when a T cell binds to such a complex on a target cell, it releases any of the cytotoxins perforin, granzyme, and granulysin. In one embodiment, through the action of perforin, granzymes enter the cytoplasm of the target cell and their serine protease function triggers a caspase cascade leading to apoptosis of said target cell. In one embodiment, apoptosis of said target cells may also be induced by cell-surface interaction between the cytotoxic T cell and the target cell, such as by a Fas-Fas ligand interaction.

The term "means for specifically targeting perilipin-1 fragment presenting adipocytes", as used herein, relates to any means which is capable of specifically targeting a perilipin-1 fragment presented on the surface of an adipocyte and which is capable of triggering the elimination of said adipocyte directly and/or indirectly. In one embodiment, said means for specifically targeting perilipin-1 fragment presenting adipocytes relates to an antigen-binding peptide capable of recognizing a perilipin-1 fragment specifically, preferably via an epitope having any of SEQ ID NOs. 1-6. In one embodiment, said means for specifically targeting perilipin-1 fragment presenting adipocytes comprises and/or induces an effector mechanism which triggers elimination of said adipocyte. In one embodiment, said means for specifically targeting perilipin-1 fragment presenting adipocytes relates to cytotoxic T cells which have a T cell receptor specific for perilipin-1, preferably specific for an epitope of perilipin-1 having any of SEQ ID NOs. 1-6. In one embodiment, said means for specifically targeting perilipin-1 fragment presenting adipocytes triggers an indirect elimination mechanism and/or a direct elimination mechanism. In one embodiment, a means for specifically targeting perilipin-1 fragment presenting adipocytes is preferably a means for specifically eliminating perilipin-1 fragment presenting adipocytes. In one embodiment, said means is capable of eliminating perilipin-1 presenting adipocytes and/or is capable of inducing an effector mechanism which eliminates perilipin-1 presenting adipocytes.

The term "specific" or "specifically binding", as used herein, means in accordance with this invention that the antibody or immune receptor fragment is capable of specifically interacting with and/or binding to a specific antigen or a set of specific antigens but does not essentially bind to other antigens. Such binding may be exemplified by the specificity of a lock-and-key-principle.

The term "nucleic acid", as used herein, relates to a nucleotide sequence, such as ribonucleic acid or deoxyribonucleic acid. In one embodiment, said nucleic acid encodes for an antigen-binding peptide in accordance with other embodiments of the present invention. In one embodiment, said nucleic acid encodes for a sequence which is at least 50 %, preferably at least 85 % identical to a nucleic acid sequence encoding for an antigen-binding peptide in accordance with other embodiments of the present invention. In one embodiment, said nucleic acid at least 50 %, preferably at least 85 % identical to a nucleic acid sequence encoding for an antigen-binding peptide results in the same peptide product as a 100 % identical sequence, due to the redundancy of the genetic code.

The term "non-human animal", as used herein, relates to an animal which is not human, preferably to a mammal.

The term "inoculating", as used herein, relates to administration of an antigen, preferably having at least one epitope having a sequence according to any of SEQ ID NOs. 1-6, for immunization and production of an antibody or antigen-binding fragment specific for said antigen.

The term "screening", as used herein, relates to analyzing certain candidates of molecules or cells with regard to their binding specificity for a certain antigen, preferably perilipin-1. The aim of such a screening is to identify molecules, such as an antigen-binding peptide, or cells, such as cytotoxic T cells, which bind to a certain antigen, preferably perilipin-1, specifically and with high affinity, preferably with an affinity of at least 10⁻⁹ M, or, with regard to TCR-derived antigen-binding fragments, preferably with an affinity of at least 10⁻⁴ M. In one embodiment, the best candidates identified by screening are subsequently amplified, for example by cell culture expansion, preferably in vitro.

The term "host cell", as used herein, relates to a cell which may be used to produce an antigen-binding peptide of the present invention. In one embodiment, said host cell allows for production of an antibody or antigen-binding fragment, such as a yeast cell or a Chinese hamster ovary (CHO) cell. In one embodiment, a host cell is transformed to comprise a nucleic acid encoding an antigen-binding peptide of the present invention and said transformed host cell is referred to as recombinant cell. In one embodiment, said host cell is an autologous or allogenic effector cell, such as a T cell or NK cell, which may be transformed into a CAR-T cell or CAR-NK cell, respectively. In one embodiment, such a cell is a genetically modified cell which comprises a nucleic acid encoding for an antigen-binding peptide specific for perilipin-1.

The term "obesity", as used herein, relates to a medical condition in which excess body fat has accumulated in a body to the extent that it may have negative effects on the health of said body. In one embodiment, a means for specifically eliminating perilipin-1 fragment presenting adipocytes, such as a perilipin-1 fragment specific antigen-binding peptide, or a perilipin-1 specific cytotoxic T cell is used to target and/or eliminate adipocytes in a patient having obesity.

The term "binding assay", as used herein, relates to an assay or an analytic procedure used to study the binding of an antigen-binding peptide to an antigen. In one embodiment, a binding assay may refer to flow-cytometry, tetramer staining, western blot, fluorescence assay, surface plasmon resonance, or other binding assays known to a person skilled in the art. In one embodiment, a binding assay (e.g. tetramer staining) may also relate to an assay used to study the binding of a cytotoxic T cell, preferably that T cell receptor of said cytotoxic T cell, to an antigen. In one embodiment, a binding assay may relate to a surface plasmon resonance measurement.

The term "K_{D}", as used herein, relates to the dissociation constant, which is the inverse of the association constant. In one embodiment, an antigen-binding peptide of the present invention binds to an antigen with an affinity (K_{D}) of at least 10⁻⁴ M. In one embodiment, said antigen-binding peptide is a peptide selected from an antibody, an antibody-derived fragment, and a non-antibody antigen-binding peptide such as an anticalin or a DARPin, and binds to an antigen with an affinity (K_{D}) of at least 10⁻⁷ M, preferably of at least 10⁻⁹ M, more preferably of at least 10⁻¹¹ M. In one embodiment, said antigen-binding peptide is a TCR-derived antigen-binding peptide and binds to an antigen and/or an MHC-antigen-complex with an affinity (K_{D}) of at least 10⁻⁴ M. In one embodiment, the term "an affinity (K_{D}) of at least 10⁻⁴ M" is meant to refer to a K_{D} value that is 10⁻⁴ M or smaller, such as 10⁻⁵ M, 10⁻⁶ M, or 10⁻⁷ M.

The term "monoclonal antibody" or "mAB", as used herein, relates to an antibody which is obtained from a population of substantially identical antibodies based on their amino acid sequence. Monoclonal antibodies are typically highly specific. Furthermore, in contrast to a polyclonal antibody preparation which typically includes different antibodies directed against different epitopes of an antigen, a mAb is typically directed against a single epitope on the antigen. In addition to their specificity, mAbs are advantageous in that they can be synthesized by cell culture, such as by a hybridoma cell culture or recombinant host cells uncontaminated by other immunoglobulins. In one embodiment, a mAb relates to a chimeric, a humanized or a human antibody or an antibody fragment. In one embodiment, a monoclonal antibody may be an optimized monoclonal antibody having an Fc region which is engineered to have improved binding properties to be targeted by an effector cell. In one embodiment, said optimized monoclonal antibody may be engineered by specific amino acid exchanges in the amino acid sequence of the Fc region. In one embodiment, said optimized monoclonal antibody may also be optimized to have an improved serum half-life, for example by modification of a CH3 region of an IgG.

The term "human antibody", as used herein, relates to an antibody which only comprises human sequences, or a fragment thereof. In one embodiment, a human antibody is produced by a display technique, such as by phage display or ribosome display.

The term "humanized antibody", as used herein, relates to an immunoglobulin chain or fragment thereof, such as a Fab fragment, a Fab' fragment, a F(ab')₂ fragment, a Fv fragment, or an other antigen-binding fragment of an antibody, which contain a sequence from non-human immunoglobulin. In one embodiment, a humanized antibody is a human antibody in which certain CDR residues are replaced by CDR residues from a non-human having desired specificity and affinity properties.

The term "chimeric-human antibody", as used herein, relates to an artificial antibody which has been designed by genetic engineering or protein engineering by combining sequences of different species, such as mouse and human. In one embodiment, a human-chimeric antibody is a hybrid protein of the antigen-binding domain from a mouse antibody and the constant domain of a human antibody.

The term "substantially intact antibody", as used herein, relates to an antibody which is not fragmented, truncated or otherwise shortened, and which has retained its capability of specifically binding to an antigen.

The term "cross-react", as used herein, relates to off-target binding to a structure which is not the target structure. In one embodiment, "cross-reacting" relates to binding of an antibody, an antigen-binding fragment, or a cytotoxic T cell, to a protein which is not perilipin-1, such as to perilipin-2, -3, -4, or -5.

The term "(%) sequence identity", as used herein, relates to the percentage of pair-wise identical residues with regard to a homology alignment of a sequence of a polypeptide/nucleic acid of the present invention with a sequence in question.

The term "patient", as used herein, may relate to a human or an animal. A blood sample for obtaining T cells and/or antibodies may be an autologous sample or an allogenic sample.

The term "recombinant antigen-binding peptide library", as used herein, relates to a pool of antigen-binding peptides produced by protein engineering technology methodologies known to a person skilled in the art. For example, such a pool of antigen-binding peptides may be produced and screened by phage display, yeast display, mRNA display or ribosome display. Display libraries display single-chain variable-domain antigen-binding fragments such as scFvs or Fab fragments, and contain the encoding DNA or RNA. The genetic diversity of these libraries is commonly generated by cloning the repertoire of the immunoglobulin heavy-chain and light-chain variable gene segments from naive or immunized individuals, or by using synthetic DNA to randomize the antigen-binding fragments, or by a combination of these approaches, optionally in combination with error-prone PCR. In one embodiment, antigen-binding peptides may be optimized to enhance an effector function and/or half-life of said antigen-binding peptide.

The term "optimized", as used herein, relates to enhanced properties of a peptide, which has been modified by protein engineering compared to said peptide in unmodified form. In one embodiment, an antigen-binding peptide is optimized with regard to the strength and/or the type of effector functions it evokes by means of an effector system, e.g. by designing said antigen-binding peptide to comprise an Fc region which is recognized by an Fc receptor of a macrophage, a monocyte, a neutrophil, a NK cell, or another effector cell. In one embodiment, said Fc region is optimized by genetic engineering to contain amino acid exchanges and/or an altered glycosylation compared to an unmodified Fc region. In one embodiment, the serum half-life of an antigen-binding peptide is increased by designing said peptide to comprise a CH3 region of an IgG domain increasing half-life or by modification of said antigen-binding peptide with other half-life increasing molecules.

### BRIEF DESCRIPTION OF THE FIGURES

The present invention is now further described by reference to the following figures.

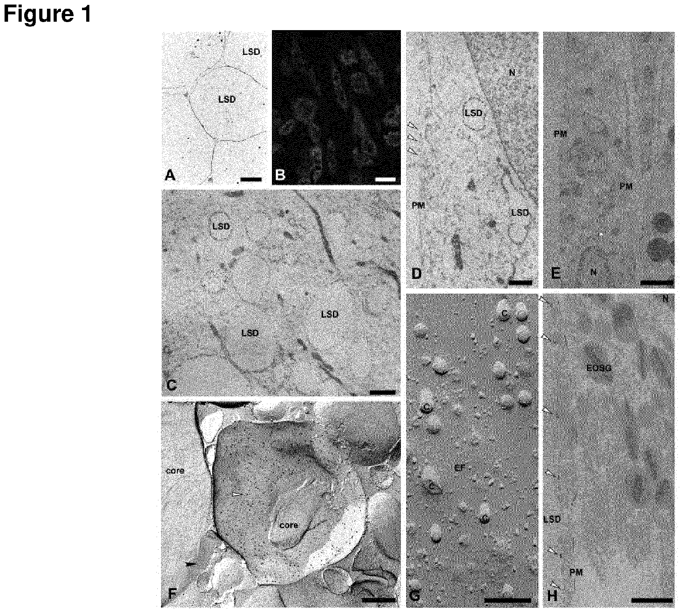

**Figure 1**: *Labeling of the membrane of lipid droplets with the IgG autoantibody from a patient with Lawrence Syndrome*/*AGL*.
   With the exception of the fluorescence labeling visualisation was accomplished using 10 nm gold particles in electron microscopy (EM) and with 1 nm gold in light microscopy (LM).
   **A**) Light microscopy of univacuolar fat cells of human white adipose tissue. Silver-enhanced gold particles only appear at the membranes of fat vacuoles, which cannot be distinguished from the plasma membrane at this magnification. The loose connective tissue between the fat cells is free of staining. Bar 25 µm.
   **B**) Fluorescence microscopy of multivacuolar 3T3-L1 cells. Labeling was visualized by the cyanine dye Cy3. Bar 50 µm.
   **C, D**) Transmission electron microscopy (TEM) of 3T3-L1 cells. The surface of lipid storage droplets is tightly labeled with gold particles. There are only sporadic gold particles at the plasma membrane (white arrowheads), whereas no gold particles appear at the nuclear envelope. Bar 1 µm.
   **E**) There is no labeling in 3T3-L1 cells at the preadipocyte stage. Bar 1 µm.
   **F**) Freeze-fracture replica of a 3T3-L1 cell. The gold label appears at the phospholipid layer (P-face, white arrowhead). There is no labeling at the convex inner droplet surface (E-face equivalent, black arrowhead) and in the core of the LSD. Bar 0.3 µm.
   **G**) Freeze-fracture replica of the exoplasmic fracture face of the plasma membrane of a 3T3-L1 cell characterized by elevated caveolae. Gold particles are not visible. Bar 0.2 µm.
   **H**) TEM of mouse adipose tissue. Gold particles are deposited at the surface of the LSD (white arrowheads). Dashed line indicates adipocyte plasma membrane. In the neighborhood there is a tissue eosinophil granulocyte.
      C, caveolae; core, lipid core of LSDs; EF, exoplasmic fracture face; EOSG, eosinophil granule; LSD, lipid storage droplet; N, nucleus; PM, plasma membrane. Bar 0.5 µm. Images shown are representative for at least 3 independent experiments.

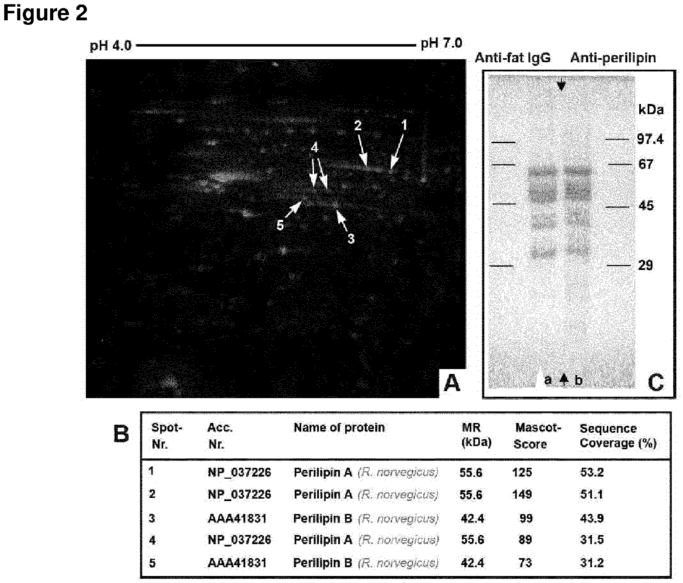

**Figure 2****:** *The human anti-fat autoantibody reacts with multiple Perilipin-1 variants.*
   **A**) Two-dimensional separation of proteins of rat fat cell membranes. Proteins were separated in a linear pH gradient IPG strip (pH 4-7) in the first dimension. In the second dimension proteins were separated using 10% (w/v) homogeneous SDS PAGE gels. In the overlay spots detected by Coomassie staining are shown in green. Spots detected by anti-fat autoantibody on a parallel blot are color coded in red. Immunoreactive spots are identified by their yellow color (marked with arrows). Spot numbers refer to the identifications listed in table B.
   **B**) Summary of the peptide mass fingerprint (PMF) analysis of protein spots. Three out of five analyzed spots were clearly identified as Perilipin A (spots 1, 2 and 4) and two spots as Perilipin B (spots 4 and 5), respectively, which result from differential splicing of the Perilipin-1 transcripts. Acc.-Nr. is the sequence identification number in GenBank. Theoretical molecular masses denoted in the fourth column were calculated from the amino acid sequence. PMF spectra were submitted for identification using Mascot interface (MASCOT 2.1.03) and Mascot scores are depicted (P=0.05).
   C) Comparison of the human anti-fat IgG with a commercial anti-Perilipin-1 antibody (Affinity BioReagents, Golden, CO, USA). 10 µg of membrane proteins were subjected to SDS-PAGE and immunoblotting was performed after cutting the PVDF membrane between two lanes of membrane proteins (↓). Incubation with the patient's autoantibody and with anti-Perilipin-1 antibody demonstrates an identical pattern of positive bands.

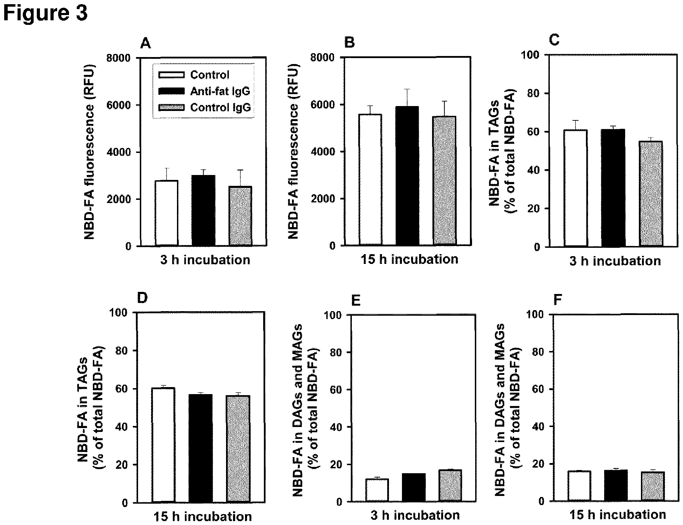

**Figure 3****:** *The autoantibody does not interfere with homeostasis of lipid storage in differentiated human preadipocytes.*
   Human preadipocytes were induced for adipose conversion and allowed to accumulate triacylglycerols over 14 days. These cells were loaded with a fluorescently labeled fatty-acid (NBD-FA) for 3 h in the presence of 10 nM insulin. After washing to eliminate excess NBD-FA the cells were incubated for an additional 3 h (A, C, E) or 15 h (B, D, F) in the absence of antibodies (white bars) or in the presence of anti-fat IgG (black bars) or control IgG (grey bars), respectively, in the presence of 10 nM insulin. Cells were harvested, total lipids were extracted and aliquots analyzed by thin-layer chromatography with subsequent quantification of labeled intracellular lipids by fluorescence scanning. The results shown are means ± SEM of 4 experiments.
   **A, B**) Anti-fat IgG does not interfere with accumulation of the NBD-FA in the total cellular lipid pool, presented as relative fluorescence units (RFU).
   **C, D**) The autoantibody has no effect on the distribution of incorporated NBD-FA between the pools of storage triacylglycerols
   **E, F**) The autoantibody has no effect on the distribution of incorporated NBD-FA between the pools of intermediates of lipid synthesis and degradation, diacylglycerols (DAGs) and monoacylglycerols (MAGs).

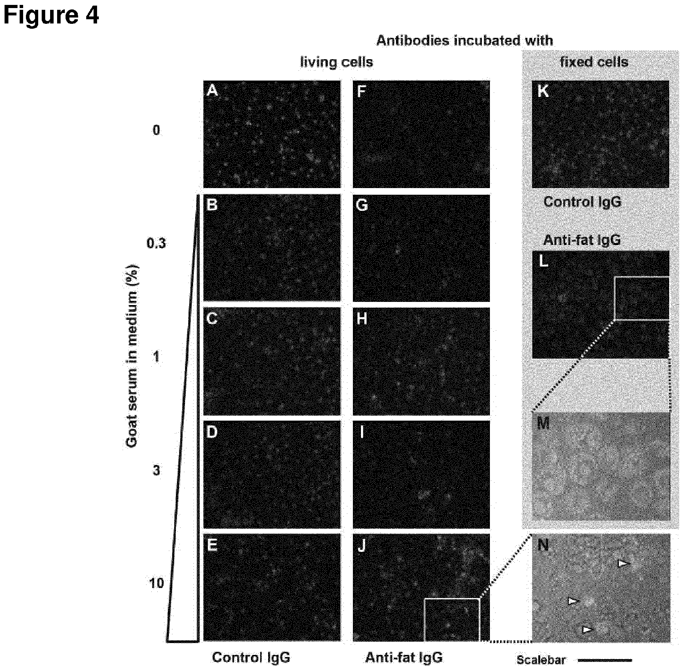

**Figure 4****.** *The anti-fat autoantibody accumulates in cells with atypical morphology (dead cells) in the presence of serum.*
   Exposure of 3T3-L1- adipocytes to neither control IgG nor anti-fat IgG in serum-free culture medium resulted in almost undetectable labeling of the cells, when the cells were washed extensively before fixation and immunofluorescence detection (**A, F**). However, anti-fat IgG (**G-J**) but not control IgG (**B-E**) resulted in immunoreactivity of a significant proportion of cells in the presence of goat serum (not heat-inactivated). This effect was strongly dose-dependent. Closer examination revealed that under these conditions anti-fat IgG did not visibly accumulate on the surface of lipid droplets (**N**), but in cells with atypical morphology (arrowheads; staining not associated with lipid droplets, condensed chromatin), which are only rarely found in normal cultures of 3T3-L1-adipocytes. Note the typical staining pattern of lipid droplets around the nucleus for cells fixed and stained according to the normal protocol (**L, M**). (**K**) No immunoreactivity in fixed cells with control IgG. (**A-J, N**) Live cells incubated with primary antibodies, (**K-M**) fixed cells incubated with primary antibodies. (**M**, **N**) Overlay of the indicated areas of the fluorescence images (**J** and **L**, respectively) and the corresponding phase contrast images; red channel set to two-fold intensity for better visibility in the overlay image. Scalebar: 250 µm (**A-L**) or 100 µm (**M-N**).

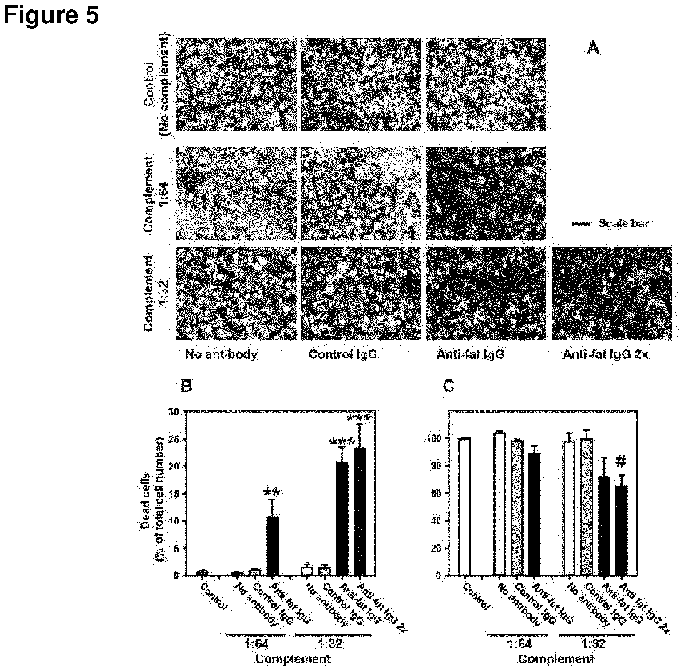

**Figure 5****.** *The anti-fat autoantibody triggers complement dependent cell-death in 3T3-L1-cells.*
   3T3-L1-adipocytes were generated in a serum-free differentiation system and allowed to accumulate lipids until day 8 after induction of differentiation. Cells were pre-incubated with anti-fat IgG or control IgG for 40 min as indicated. Then, medium without (control) or with rabbit complement (final dilution either 1:64 or 1:32) was added as noted in the figure. In the presence of complement 1:32 anti-fat IgG was additionally tested in twice the concentration normally used (anti-fat IgG 2x).
   **A**) Cells were fixed and stained with Fluoro Quench AO/EB reagent, which results in green staining of living cells and in orange staining of dying and dead cells (in flat adipocytes the lipid droplets appear unstained, whereas in rounded adipocytes they are merely visible). Scale bar 100 µm for all images.
   **B**) The quantification of 3 independent experiments indicates significant numbers of dead cells in the presence of anti-fat IgG plus complement, but not under control conditions. Significant differences versus all respective controls were identified by ANOVA (P<0.005) with Student-Newman-Keuls all pairwise multiple comparisons; **, P<0.01; ***, P<0.001.
   **C**) Percentage of viable cells in relation to corresponding untreated controls after 1 hour (Student's t-test; #, P<0.05).

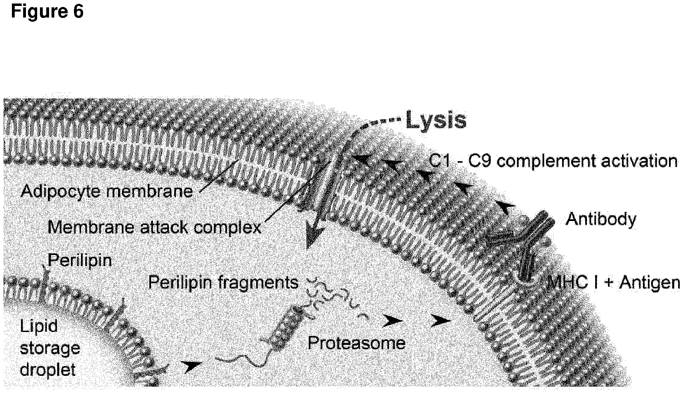

**Figure 6****.** *Scheme of perilipin-1-dependent antibody-induced complement activation.*
   Mechanism of action of an induced complement reaction against adipocytes which is mediated by anti-perilipin-1 antibodies. Perilipin-1 peptides are degraded by the proteasome and the resulting perilipin-1 fragments are presented by an MHC on the cell surface of adipocytes. Antibodies targeting a peptide sequence, i.e. an epitope having any of SEQ ID NOs. 1-6, as disclosed herein, specifically target adipocytes and allow for specific elimination of adipocytes by complement activation (indirect elimination mechanism). Alternatively, cytotoxic T cells specific for at least one perilipin-1 epitope may eliminate adipocytes presenting said perilipin-1 by an indirect elimination mechanism.
   In addition, other effector systems which may be activated by antibodies binding to perilipin-1 fragment-loaded MHC may employ a direct elimination mechanism. These effector systems, which are activated by Fc-receptor mediated binding to the said adipocyte bound antibodies, may be macrophages or neutrophils (neutrophilic polymorphonuclear granulocytes).

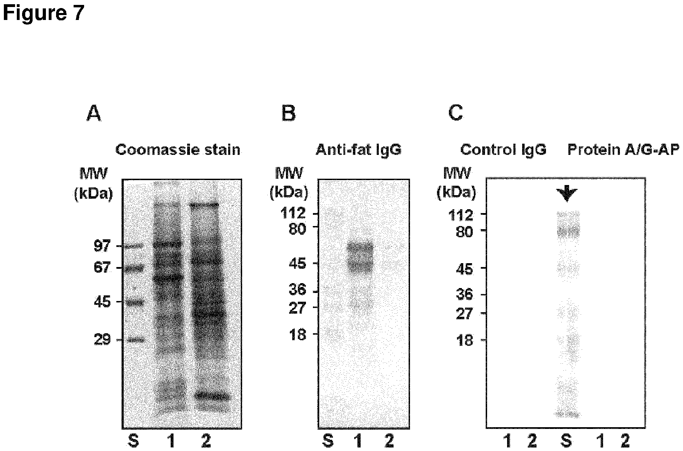

**Figure 7****.** *The anti-fat autoantibody binds to lipid associated proteins.*
   Lipid associated (1) and cytosolic proteins (2) from isolated fat cells of rat fat pads were separated by SDS-PAGE and either stained with Coomassie blue (**A**) or transferred to a PVDF membrane (**B**). The anti-fat IgG reacts with two major and several minor bands of the preparation containing lipid associated proteins. (**C**) A control membrane was cut along the molecular weight standard lane (↓) and incubated with either a control serum and subsequently with protein A/G-AP (Control IgG) or with protein A/G-AP alone (Protein A/G-AP), indicating that recognition of bands in (**B**) was specific for the anti-fat antibody. S, molecular weight standard. Data are representative for 5 experiments with rat fat pads and similar results were obtained with in vitro differentiated human adipocytes and mouse 3T3-L1-cells.

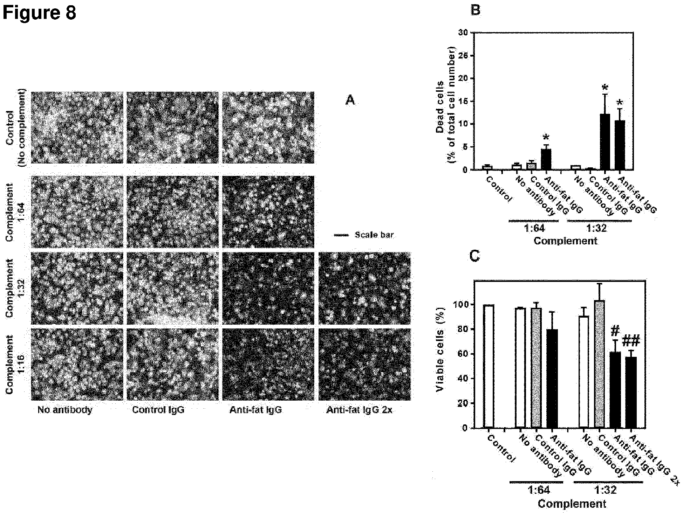

**Figure 8****.** *Only a fraction of cells is susceptible to autoantibody triggered cell death.*
   Cells were treated in parallel to the experiments summarized in figure 5, but incubations lasted 24 hours. In addition, data obtained with a higher complement concentration (final dilution 1:16) are shown (**A**). The quantification of 3 independent experiments indicates significant loss of cells in the presence of anti-fat IgG plus complement, but not under control conditions. When comparing numbers for dead cells (**B**) and viable cells (**C**), there is a remarkable difference due to detachment of dead cells. (**B**) Significant differences versus all respective controls were identified by ANOVA (p=0.016) with Student-Newman-Keuls all pairwise multiple comparisons; *, P<0.05. (**C**) Percentage of viable cells in relation to corresponding untreated controls after 24 hours (Student's t-test; #, P<0.05; ##, P<0.005).

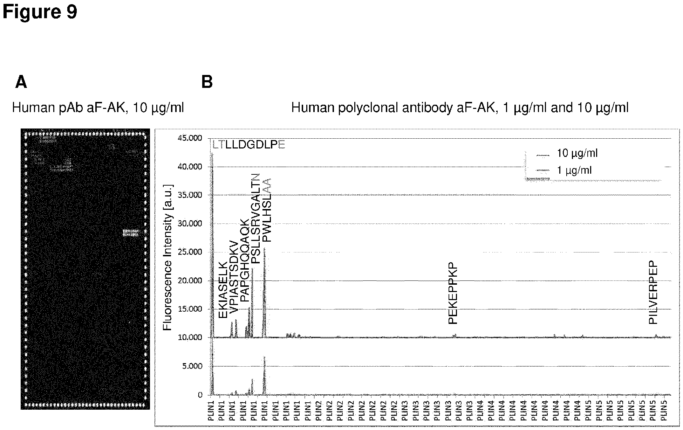

**Figure 9****.** *Peptide array for perilipin-1 epitopes.*
   Perilipin-1 epitopes having SEQ ID NOs. 1-6 were identified using peptide arrays (PEPperPRINT, PEPperMAP) with perilipin-1 autoantibodies. Sequences of human perilipins 1-5 were elongated with neutral linker sequences at the N- and C-terminal ends to prevent shortened peptides. The elongated antigen sequences were translated into peptide sequences having 15 amino acids each, wherein said peptide sequences had an overlap of 14 amino acids each. The thus produced peptide array was screened with the perilipin-1 autoantibody described in the previous examples. The antibody specificity was verified by screening all possible 15mer peptide sequences of perilipin-1 related proteins perilipin-2, -3, -4- and 5, which are involved in different functions of lipid storage and/or in other cells. Accordingly, cross-reactivity with other members of the perilipin family was excluded.
   **A**) Analysis of a peptide array using a human anti-perilipin-1 autoantibody to identify perilipin-1 epitopes. Only perilipin-1 peptides are bound specifically and with high affinity.
   **B**) Quantification of the antibody binding. Six peptides were bound with high affinity. The binding of the peptide depicted on the right (PEKEPPKP from perilipin-3 and PILVERPEP from perilipin-5) was negligible.
      The plurality of the identified peptides (epitopes) is highly conserved in the mouse sequence and human sequence.

### EXAMPLES

### Example 1: Isolation of autoantibodies targeting perilipin-1.

Written informed consent was obtained from all donors of tissue samples according to a protocol, which was approved by the ethics committee of the Jena University Hospital. The stromal vascular fractions of cells from adipose tissue samples (referred to as human preadipocytes) were isolated and cultured in medium 199 containing 10 % (v/v) fetal calf serum until confluence. 3T3-L1 cells were obtained from the ATCC and were maintained. All experiments involving differentiation or stimulation of 3T3-L1-cells were done in a serum-free medium consisting of DMEM and Ham's F12 medium in a ratio of 3:1 (without phenol-red and with 7.5 mM HEPES, pH 7.2), which was supplemented with gentamycin (40 µg/ml), transferrin (2 µg/ml), pantothenate (17 µM), biotin (1 µM), and insulin (1 nM). For adipose conversion of 3T3-L1-cells this medium was further supplemented with fetuin (300 µg/ml). Except indicated otherwise, all experiments involving anti-fat IgG and live cells (including the complement activation assay) were done in the absence of serum. Immunoglobulin G (IgG) from 5 ml patients' serum (anti-fat IgG) or control serum (control IgG) was purified on thiophilic agarose (AFFI-TTM; Kem-En-Tec, Taastrup, Denmark) according to manufacturers' instructions.

### Example 2: Preparation and analysis of fat cell lipid associated proteins.

Male Wistar rats were used. The fat cell suspension in PBS was extensively shaken with half the volume chloroform. Centrifugation (2500×g) at 25°C for 5 min separated lipids dissolved in chloroform from supernatant cytoplasmic proteins and lipid associated proteins in the fat cake at the interface of the organic and aqueous phases. Extraction of the fat cake by chloroform was repeated twice. Proteins of the supernatant were precipitated with acetone at - 20°C for 1 h. Lipid associated proteins were suspended by vortexing in PBS. Residual chloroform in the final pellet was evaporated in vacuo. 100 µg lipid associated proteins or cytoplasmic proteins from fat cells were solubilized with 100 µl sample buffer containing 8 M urea. 10 µl samples were separated on minigels at 4°C. Gels were either fixed and stained with Coomassie blue or proteins were transferred onto PVDF membranes (Immobilon-P, 0.45 µm, Millipore, Bedford) for 2 h at 1 mA/cm2 at 8°C using a semi-dry blotting unit. After staining with Ponceau S for 2 min membranes were washed with 0.1 N NaOH, rinsed with water, blocked overnight in 0.2 % I-block (TROPIX, Bedford, MA) in Tris buffered saline-Tween 20 (TBS-T), and incubated with the primary antibodies for 1 h: anti-fat IgG and control-IgG were used at a dilution of 1:1000, polyclonal rabbit anti-Perilipin-1 at 1:1250. The protein G-alkaline phosphatase (AP) was diluted 1:5000. Anti-rabbit IgG-AP was diluted 1:1250. Blots were developed with the Nitro blue tetrazolium method. Proteomic analysis of fat cake proteins was performed.

### Example 3: Assessment of storage lipid metabolism.

Confluent monolayers of human preadipocytes were induced for adipose conversion in serum-free medium containing 1 µM insulin, 1 µM cortisol, 500 µM 3-isobutyl-1-methylxanthine, and 1 µM rosiglitazone for 8 days and subsequently allowed to accumulate triacylglycerols in the presence of insulin alone until day 14. The cellular pool of fatty acid containing lipids, mainly triacylglycerols, was labeled with the fluorescent fatty acid analog 12-(N-(7-nitrobenz-2-oxa-1,3-diazol-4-yl)amino)dodecanoic acid (NBD-FA) and analyzed by thin-layer chromatography and fluorescence imaging. Briefly, labeling was done in serum-free medium containing 1 % (w/v) fatty acid free bovine serum albumin and 100 µM NBD-FA. After washing to remove excess NBD-FA the cells were incubated in the absence or presence of antibodies for various times. Incubations were terminated by removal of media and immediate extraction of lipids with 2-propanol.

### Example 4: Labeling of the membrane of lipid droplets with the IgG autoantibody from a patient with lipoatrophy.

The serum containing autoantibodies against adipose tissue from a patient with AGL was used to identify the target autoantigen. The purified IgG-fraction of the autoimmune serum (anti-fat IgG) exclusively labeled adipose tissue, but not other tissues tested. Labeling was confined to adipocytes in tissue sections analyzed by immunohistochemistry (Fig. 1 A). Besides human univacuolar adipocytes, anti-fat IgG stained adipocytes from all other mammalians tested, but not from chicken and carp (data not shown). Immunofluorescent staining of in vitro differentiated 3T3-L1 mouse adipocytes containing multiple lipid storage droplets (LSDs) (Fig. 1 B) suggested a specific reaction of the anti-fat IgG with the surface of LSDs. This specific labeling of the LSD surface was confirmed by transmission electron microscopy on immuno-gold labeled ultrathin sections of differentiated 3T3-L1-cells (Fig. 1 C). Adipocyte plasma membranes were labeled only sporadically (Fig. 1 D), undifferentiated cells showed no labeling at all (Fig. 1 E), suggesting that the expression of the autoantigen is almost exclusively limited to the LSDs of adipocytes. These findings were confirmed in ultrathin sections from mouse adipose tissue (Fig. 1 H).

### Example 5: The anti-fat autoantibody reacts with lipid associated proteins identified as multiple Perilipin-1 variants.

Isolated fat cells of rat epididymal fat pads were used to prepare a delipidized fraction of lipid-associated proteins and a cytosolic fraction. In western blot experiments with the anti-fat IgG a characteristic pattern of two major and several minor bands was found almost exclusively in the fraction of lipid-associated proteins (Fig. 7 B). As neither IgG of a healthy control nor protein A/G-AP alone reacted with these bands (Fig. 7 C), recognition of this band pattern is specific for the anti-fat autoantibody. Thus, fractionation of rat adipocytes confirmed the results of the microscopic analyses. Having confirmed that the anti-fat IgG specifically labels (a) putative autoantigen(s) from adipocytes, we set out for identifying the autoantigen(s). After separation of the lipid associated protein fraction by two-dimensional electrophoresis several groups of immuno-reactive spots were identified by the anti-fat IgG (Fig. 2 A). These spots were isolated, subjected to tryptic digestion and peptides were subsequently analyzed by MALDI-TOF-MS. Searches in the NCBI protein data base using the mass spectrometry data identified all immuno-reactive spots as variants of Perilipin-1 (Fig. 2 B). Direct comparison of the anti-fat IgG and a commercial anti-Perilipin-1 antibody revealed identical staining patterns in western analysis, confirming Perilipin-1 as an adipocyte-specific autoantigen in AGL (Fig. 2 C).

### Example 6: The anti-fat autoantibody accumulates in cells with atypical morphology in the presence of serum.

In order to obtain information regarding the mechanism by which the anti-fat IgG causes adipocyte loss, it was assessed whether the anti-fat IgG disturbs storage lipid metabolism in intact cells. Therefore, cells were labeled with a fluorescent fatty-acid (NBD-FA), which is a substrate for both, lipogenic and lipolytic, enzymes. Neither control IgG nor the anti-fat IgG did alter the total amount of labeled lipids during 3 h or 15 h incubations (Fig. 3A, B). More specific examination of storage triacylglycerols (Fig. 3C, D) and intermediary products of lipolysis, diacylglycerols and monoacylglycerols, (Fig. 3E, F) also revealed no effect of the anti-fat IgG in in vitro differentiated human adipocytes. Thus, in accordance with the almost exclusive localization of Perilipin-1 at the surface of LSDs (Fig. 1) it is unlikely that interference of the anti-fat IgG with the normal function of Perilipin-1 causes adipocyte loss. However, when living 3T3-L1-adipocytes were incubated with antibodies in the absence or presence of increasing amounts of goat serum, a dose dependent increase of the number of labeled cells could be detected when anti-fat IgG was present (Fig. 4F-J), but not when control IgG was used (Fig. 4A-E). Examination of the stained cells revealed association of the anti-fat IgG with cells presenting atypical morphology (dying or dead cells) (Fig. 4J, N). The serum dose dependent increase of such atypical cells indicated that serum factor(s) might cooperate with anti-fat IgG to induce cell-death. This is the classical hallmark of the complement system.

### Example 7: Complement activation assay.

3T3-L1-cells were induced for differentiation under serum-free conditions in 96-well plates and allowed to accumulate lipids until day 8. Then cells were pre-incubated in serum-free medium without or with antibodies in a total volume of 60 µl for 40 min, thereafter 20 µl of various dilutions of rabbit complement (BAG, Lich, Germany) in serum-free medium were added. To terminate the reactions, 40 µl Fluoro Quench AO/EB reagent (OneLambda, Canoga Park, USA) were added. 15 min later, the fluorescence images were captured using a FITC-filter (longpass emission). Complement dilutions were prepared fresh and checked for activity before each individual experiment.

### Example 8: The anti-fat autoantibody triggers complement dependent cell-death in 3T3-L1-cells.

It was tested whether the anti-fat IgG directs a complement-mediated reaction towards intact adipocytes. Complement alone or in the presence of control IgG was without effect on staining of dead or viable cells, respectively (Fig. 5). In contrast, within one hour anti-fat IgG induced a massive increase of the dead cell number in the presence of complement (Fig. 5A, B). A higher complement concentration led to an additional increase in the number of dead cells, which was mirrored by a significant loss of viable cells (Fig. 5 C). Extension of the incubation times to 24 hours did not affect cell viability or dead cell numbers in the absence of anti-fat IgG but induced an even more pronounced loss of viable cells in the presence of anti-fat IgG (Fig. 8). Obviously, the low density of epitope(s) targeted by the anti-Perilipin-1 autoantibody in adipocyte plasma membranes (see Fig. 1 D) was sufficient to trigger complement mediated cell death. Interestingly, after treatment with complement plus anti-fat IgG the remaining viable cell populations consisted mainly of preadipocytes lacking LSDs and Perilipin-1, again indicating the necessity of Perilipin-1 expression in target cells of the anti-fat IgG induced complement reaction.

### Example 9: Anti-perilipin-1 autoantibodies trigger complement-mediated destruction of adipocytes in acquired generalized lipodystrophy.

Generalized lipodystrophies are associated with severe metabolic complications. Familial types were tracked down to defects of key genes of adipocyte function. Pathogenesis of the acquired lipodystrophies is less understood, but often accompanied by chronic infections or autoimmune markers. From serum of a patient with AGL we isolated autoantibodies against adipose tissue as described in the previous examples. The immunoglobulin G-fraction of patient serum, but not that of normal sera, specifically stained the lipid accumulating adipocytes in tissue sections and in vitro, indicating a direct immune reaction against adipocytes involved in the etiology of AGL. The lipodystrophy-associated autoantibodies react with an intracellular autoantigen, which is exclusively expressed in adipocytes, located at the surface of lipid storage droplets, and said antigen was identified as Perilipin-1 by two-dimensional electrophoresis coupled with mass spectrometry. The autoantibodies did not alter cellular lipid homeostasis and did not enter intact cells. In the presence of serum factors or complement, however, the autoantibodies induced cell death in cultured adipocytes. The present invention discloses a cellular target, namely Perilipin-1, which has a key role in the selective loss of adipocytes in AGL via a complement mediated reaction.

The present inventors disclose that lipodystrophy-associated autoantibodies react with an autoantigen, which is exclusively expressed in adipocytes, located at the surface of lipid storage droplets, identified as perilipin-1. The autoantibodies do not alter cellular lipid homeostasis and do not enter intact cells. In the presence of serum factors or complement, however, the autoantibodies induce cell death in cultured adipocytes. The anti-perilipin-1 autoantibodies are responsible for targeting a complement reaction against adipocytes.

### REFERENCES

[1] Greenberg AS, Egan JJ, Wek SA, Moos MC, Jr., Londos C, Kimmel AR. Isolation of cDNAs for perilipins A and B: sequence and expression of lipid droplet associated proteins of adipocytes. Proc Natl Acad Sci USA, 1993;90:12035-9.
[2] Hübler A, Abendroth K, Keiner T, Stocker W, Kauf E, Hein G et al. Dysregulation of insulin-like growth factors in a case of generalized acquired lipoatrophic diabetes mellitus (Lawrence Syndrome) connected with autoantibodies against adipocyte membranes. Exp Clin Endocrinol Diabetes, 1998;106:79-84.
[3] Corvillo F, Aparicio V, Lopez-Lera A, Garrido S, Araujo-Vilar D, et al. Autoantibodies against perilipin 1 as a cause of acquired generalized lipodystrophy. Front Immunol, 2018; 9:2142.
[4] Misra A, Garg A. Clinical features and metabolic derangements in acquired generalized lipodystrophy: case reports and review of the literature. Medicine (Baltimore), 2003;82:129-46.

The features of the present invention disclosed in the specification, the claims, and/or in the accompanying figures may, both separately and in any combination thereof, be material for realizing the invention in various forms thereof.

## Claims

1. An antigen-binding peptide specifically binding to at least one perilipin-1 epitope selected from the following amino acid sequences: LTLLDGDLPE (SEQ ID NO. 1), EKIASELK (SEQ ID NO. 2), VPIASTSDKV (SEQ ID NO. 3), PAPGHQQAQK (SEQ ID NO. 4), PSLLSRVGALTN (SEQ ID NO. 5), and PWLHSLAA (SEQ ID NO. 6).

2. The antigen-binding peptide according to claim 1, wherein antigen-binding peptide binds to more than one of SEQ ID NO. 1-6.

3. The antigen-binding peptide according to claim 1 or 2, wherein said perilipin-1 epitope is a human perilipin-1 epitope.

4. The antigen-binding peptide according to any of the foregoing claims, wherein said antigen-binding peptide is not a perilipin-1 targeting autoantibody of a human patient having lipoatrophy, and wherein preferably said antigen-binding peptide is not a perilipin-1 targeting anti-PLINl antibody directed to the peptide spanning Thr8-Ala145 having catalog # AF6615 from manufacturer R&D.

5. The antigen-binding peptide according to any of the foregoing claims, wherein said antigen-binding peptide competes for binding with a perilipin-1 targeting autoantibody of a human patient having lipoatrophy.

6. The antigen-binding peptide according to any of the foregoing claims, wherein said antigen-binding peptide binds to perilipin-1 in a binding assay with an affinity (K_{D}) of at least 10⁻⁴ M.

7. The antigen-binding peptide according to any of the foregoing claims, wherein said antigen-binding peptide is a monoclonal antibody.

8. The antigen-binding peptide according to any of the foregoing claims, wherein said antigen-binding peptide is a human antibody, a humanised antibody, or a chimeric-human antibody.

9. The antigen-binding peptide according to any of the foregoing claims, wherein said antigen-binding peptide is an intact antibody, a substantially intact antibody, a Fab fragment, a F(ab')₂ fragment, or a single chain Fv fragment.

10. The antigen-binding peptide according to any of the foregoing claims, wherein said antigen-binding peptide is capable of binding to a perilipin-1 fragment presented on an adipocyte, and wherein said binding of said antigen-binding peptide to a perilipin-1 fragment on said adipocyte activates an immune effector system to target and/or eliminate said adipocyte.

11. The antigen-binding peptide according to any of the foregoing claims, wherein said antigen-binding peptide comprises an Fc region, preferably an Fc region which is optimized by protein engineering for higher binding affinity to an effector cell compared to a binding affinity of an Fc region which has not been optimized.

12. The antigen-binding peptide, wherein said antigen-binding peptide does not cross-react with any other perilipin protein, such as perilipin-2 -3, -4, or -5.

13. An antigen-binding peptide as defined in any of the foregoing claims for use in the prevention or treatment of obesity.

14. A bispecific antigen-binding peptide comprising
(i) a first binding site specifically binding to a perilipin-1 epitope having any of SEQ ID NO. 1-6; and
(ii) a second binding site.

15. The bispecific antigen-binding peptide according to claim 14, wherein said second binding site binds to a perilipin-1 epitope having any of SEQ ID NO. 1-6, wherein said epitope is equal to or different from the epitope of said first binding site; to a white adipocyte-specific surface marker, for example the neutral amino acid transporter ASC-1; or to an immune cell specific receptor molecule, such as a T cell specific receptor molecule or a natural killer cell specific receptor molecule.

16. A bispecific antigen-binding peptide according to claim 14 or 15 for use in the prevention or treatment of obesity.

17. A pharmaceutical composition comprising the antigen-binding peptide according to any of claims 1-12, and/or the bispecific antigen-binding peptide according to any of claims 14-15, for use in the prevention or treatment of obesity.

18. An isolated nucleic acid encoding for an antigen-binding peptide as defined in any of claims 1-12, or encoding for a bispecific antigen-binding peptide as defined in claim 14 or 15.

19. A recombinant cell comprising a nucleic acid as defined in claim 18, wherein said recombinant cell is preferably a recombinant autologous effector cell or a recombinant host cell.

20. A method of producing a means for specifically targeting perilipin-1 fragment presenting adipocytes, comprising:
a) Inoculating a non-human animal with an antigen comprising at least one perilipin-1 specific epitope selected from SEQ ID NO. 1-6; and screening and producing an antigen-binding peptide specifically binding to the at least one perilipin-1 specific epitope from the inoculated animal, preferably by harvesting cells expressing an antigen-binding peptide from the spleen of said non-human animal, preparing a hybridoma cell therefrom, expressing said antigen-binding peptide, and screening said antigen-binding peptide expressed by said hybridoma cell to identify an antigen-binding peptide specifically binding to any of SEQ ID NO. 1-6, and isolation thereof; or
b) providing and screening a recombinant antigen-binding peptide library, such as a library of antibodies, scFv molecules, Camel antibodies, Anticalins, or DARPins, to identify an antigen-binding peptide specifically binding to any of SEQ ID NO. 1-6, and isolation thereof; or
c) providing and screening a cell pool comprising cytotoxic T cells, such as by Tetramer-staining or Dextramer-Technology, to identify a cytotoxic T cell having specificity to any of SEQ ID NO. 1-6, and isolation thereof; or
d) providing a blood sample of a patient and obtaining T cells therefrom, genetically modifying said T cells by introducing a nucleic acid as defined in claim 18, and obtaining chimeric antigen receptor expressing T cells; or
e) providing and screening a pool of T cell receptors to identify a T cell receptor having specificity to any of SEQ ID NO. 1-6, and isolation thereof.

21. The method according to claim 20, wherein said means targets perilipin-1 and is selected from an antigen-binding peptide and a cytotoxic T cell.


SEQUENCE LISTING

<110> Alytas Therapeutics GmbH

<120> Means for specifically eliminating perilipin-1 fragment presenting
adipocytes

<130> A33031EP

<160> 7

<170> BiSSAP 1.3.6

<210> 1
<211> 10
<212> PRT
<213> Homo sapiens

<220>
<223> epitope

<400> 1
Leu Thr Leu Leu Asp Gly Asp Leu Pro Glu
1               5                   10

<210> 2
<211> 8
<212> PRT
<213> Homo sapiens

<220>
<223> epitope

<400> 2
Glu Lys Ile Ala Ser Glu Leu Lys
1               5

<210> 3
<211> 10
<212> PRT
<213> Homo sapiens

<220>
<223> epitope

<400> 3
Val Pro Ile Ala Ser Thr Ser Asp Lys Val
1               5                   10

<210> 4
<211> 10
<212> PRT
<213> Homo sapiens

<220>
<223> epitope

<400> 4
Pro Ala Pro Gly His Gln Gln Ala Gln Lys

1                5                   10

<210> 5
<211> 12
<212> PRT
<213> Homo sapiens


<220>
<223> epitope

<400> 5
Pro Ser Leu Leu Ser Arg Val Gly Ala Leu Thr Asn
1               5                   10

<210> 6
<211> 8
<212> PRT
<213> Homo sapiens


<220>
<223> epitope

<400> 6
Pro Trp Leu His Ser Leu Ala Ala
1               5

<210> 7
<211> 522
<212> PRT
<213> Homo sapiens


<220>
<223> full-length human perilipin-1

<400> 7
Met Ala Val Asn Lys Gly Leu Thr Leu Leu Asp Gly Asp Leu Pro Glu
1               5                   10                  15
Gln Glu Asn Val Leu Gln Arg Val Leu Gln Leu Pro Val Val Ser Gly
            20                  25                  30
Thr Cys Glu Cys Phe Gln Lys Thr Tyr Thr Ser Thr Lys Glu Ala His
        35                  40                  45
Pro Leu Val Ala Ser Val Cys Asn Ala Tyr Glu Lys Gly Val Gln Ser
    50                  55                  60
Ala Ser Ser Leu Ala Ala Trp Ser Met Glu Pro Val Val Arg Arg Leu
65                  70                  75                  80
Ser Thr Gln Phe Thr Ala Ala Asn Glu Leu Ala Cys Arg Gly Leu Asp
                85                  90                  95
His Leu Glu Glu Lys Ile Pro Ala Leu Gln Tyr Pro Pro Glu Lys Ile
            100                 105                 110
Ala Ser Glu Leu Lys Asp Thr Ile Ser Thr Arg Leu Arg Ser Ala Arg
        115                 120                 125
Asn Ser Ile Ser Val Pro Ile Ala Ser Thr Ser Asp Lys Val Leu Gly
    130                 135                 140
Ala Ala Leu Ala Gly Cys Glu Leu Ala Trp Gly Val Ala Arg Asp Thr
145                 150                 155                 160
Ala Glu Phe Ala Ala Asn Thr Arg Ala Gly Arg Leu Ala Ser Gly Gly
                165                 170                 175
Ala Asp Leu Ala Leu Gly Ser Ile Glu Lys Val Val Glu Tyr Leu Leu
            180                 185                 190
Pro Pro Asp Lys Glu Glu Ser Ala Pro Ala Pro Gly His Gln Gln Ala

195                 200                 205
Gln Lys Ser Pro Lys Ala Lys Pro Ser Leu Leu Ser Arg Val Gly Ala
    210                 215                 220
Leu Thr Asn Thr Leu Ser Arg Tyr Thr Val Gln Thr Met Ala Arg Ala
225                 230                 235                 240
Leu Glu Gln Gly His Thr Val Ala Met Trp Ile Pro Gly Val Val Pro
                245                 250                 255
Leu Ser Ser Leu Ala Gln Trp Gly Ala Ser Val Ala Met Gln Ala Val
            260                 265                 270
Ser Arg Arg Arg Ser Glu Val Arg Val Pro Trp Leu His Ser Leu Ala
        275                 280                 285
Ala Ala Gln Glu Glu Asp His Glu Asp Gln Thr Asp Thr Glu Gly Glu
    290                 295                 300
Asp Thr Glu Glu Glu Glu Glu Leu Glu Thr Glu Glu Asn Lys Phe Ser
305                 310                 315                 320
Glu Val Ala Ala Leu Pro Gly Pro Arg Gly Leu Leu Gly Gly Val Ala
                325                 330                 335
His Thr Leu Gln Lys Thr Leu Gln Thr Thr Ile Ser Ala Val Thr Trp
            340                 345                 350
Ala Pro Ala Ala Val Leu Gly Met Ala Gly Arg Val Leu His Leu Thr
        355                 360                 365
Pro Ala Pro Ala Val Ser Ser Thr Lys Gly Arg Ala Met Ser Leu Ser
    370                 375                 380
Asp Ala Leu Lys Gly Val Thr Asp Asn Val Val Asp Thr Val Val His
385                 390                 395                 400
Tyr Val Pro Leu Pro Arg Leu Ser Leu Met Glu Pro Glu Ser Glu Phe
                405                 410                 415
Arg Asp Ile Asp Asn Pro Pro Ala Glu Val Glu Arg Arg Glu Ala Glu
            420                 425                 430
Arg Arg Ala Ser Gly Ala Pro Ser Ala Gly Pro Glu Pro Ala Pro Arg
        435                 440                 445
Leu Ala Gln Pro Arg Arg Ser Leu Arg Ser Ala Gln Ser Pro Gly Ala
    450                 455                 460
Pro Pro Gly Pro Gly Leu Glu Asp Glu Val Ala Thr Pro Ala Ala Pro
465                 470                 475                 480
Arg Pro Gly Phe Pro Ala Val Pro Arg Glu Lys Pro Lys Arg Arg Val
                485                 490                 495
Ser Asp Ser Phe Phe Arg Pro Ser Val Met Glu Pro Ile Leu Gly Arg
            500                 505                 510
Thr His Tyr Ser Gln Leu Arg Lys Lys Ser
        515                 520